# EUROPEAN PATENT APPLICATION

(11) **EP 4 710 949 A1**
(43) Date of publication of application: **18.03.2026**
(21) Application number: 24807053.4
(22) Date of filing: 01.05.2024
(51) Int. Cl.: A61K 49/00, A61K 47/10, A61K 47/20, A61K 47/26

(54) **LYOPHILIZED PREPARATION, LYOPHILIZED SOLUTION, METHODS FOR PRODUCING SAME, AND LIQUID AGENT**

(30) Priority: 12.05.2023 JP 2023079476
(71) Applicant: Goryo Chemical, Inc., Sapporo-shi, Hokkaido 060-0008 (JP)
(72) Inventor: SUZUKI, Yuki, Sapporo-shi, Hokkaido 060-0008 (JP)
(74) Representative: Müller-Boré & Partner Patentanwälte PartG mbB
(86) International application number: PCT/JP2024/016753
(87) International publication number: WO 2024/237099

(57) **Abstract**

The present invention provides: a lyophilized preparation including a compound represented by the following general formula (I) or a salt thereof; a lyophilization solution including a compound represented by the following general formula (I) or a salt thereof, a solubilizing agent, and water; methods of producing the same; and a liquid preparation obtained by reconstituting the above-mentioned lyophilized preparation.

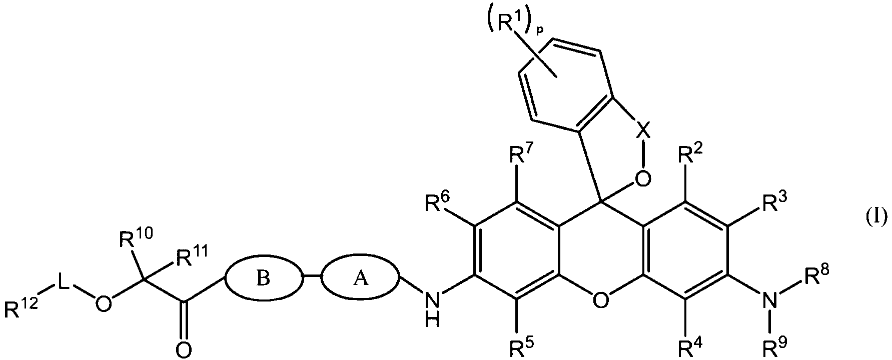

## Description

### Technical Field

The present invention relates to a lyophilized preparation, a lyophilization solution, and methods of producing the same, and a liquid preparation.

### Background Art

A calpain is a cysteine protease activated in a Ca²⁺-dependent manner, and is a modulator molecule that regulates the function and structure of a substrate protein through the limited cleavage of the protein. While the calpain is involved in various life phenomena, it has been reported that the pathogenic variation of its activity or its gene is associated with various diseases, such as a neurodegenerative disease, a heart or muscle disease, an ischemic disease, a cancer, and an eye disease. Further, the calpain is essential to the survival, infection, and the like of the pathogens of some infectious diseases, and it has been known that the calpain is associated with, for example, malaria, trypanosomiasis, or schistosomiasis. Accordingly, it is important to visualize the activity of the calpain in diagnosis of calpain-associated diseases and analysis on the disease mechanisms/drug discovery research.

A fluorescence imaging method serves as an effective method of detecting calpain activity in real time. For example, a probe obtained by bonding a peptide to hydroxymethyl rhodamine green (HMRG) serving as a fluorescent dye has been reported as means for detecting calpain activity in a live cell without relying on gene introduction (Patent Literature 1).

### Citation List

### Patent Literature

[PTL 1] WO 2022/270607 A1

### Summary of Invention

### Technical Problem

When the probe described in Patent Literature 1 is used in, for example, the diagnosis of a calpain-associated disease, it is assumed that the fluorescence imaging of calpain activity is performed by: preparing an aqueous solution of the probe; and bringing the aqueous solution into contact with an affected part through an administration route, such as injection or a spray.

However, as a result of a further investigation made by the inventors of the present invention toward the practical application of the probe described in Patent Literature 1, it has been found that it is difficult to provide an administration liquid, which contains the probe described in Patent Literature 1 at a desired concentration, at the time of clinical use because the above-mentioned probe has low water solubility, and cannot be instantly dissolved in water. In addition, it has been found that the aqueous solution of the probe described above is susceptible to improvement in terms of its content and stability of its purity.

The present invention has been made to solve the above-mentioned problems, and a primary object of the present invention is to provide a preparation, from which an administration liquid containing the above-mentioned probe at a practical concentration can be prepared, and which is excellent in the content of the probe and stability of its purity.

### Solution to Problem

[1] According to one aspect of the present invention, there is provided a lyophilized preparation, including a compound represented by the following general formula (I) or a salt thereof: 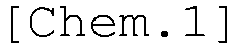 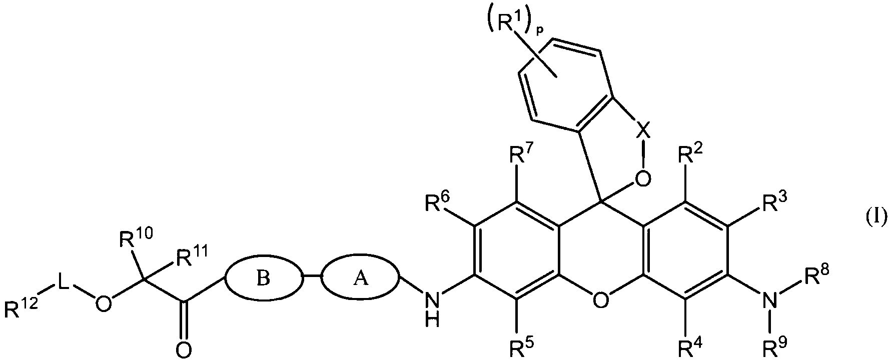 where:
   A and B each independently represent an amino acid residue, and the amino acid residues may be identical to or different from each other,
      where a bond between A and NH bonded to A is an amide bond between a C-terminal of A and the NH, A and B are bonded through an amide bond, and a bond between B and a carbonyl group (C=O) bonded to B is an amide bond between an N-terminal of B and the carbonyl group (C=O);
   R¹s may be identical to or different from each other, and each represent a substituent bonded to a benzene ring;
   "p" represents an integer of from 0 to 4;
   R², R³, R⁴, R⁵, R⁶, and R⁷ each independently represent a hydrogen atom, a hydroxyl group (OH group), a halogen atom, or a linear, branched, or cyclic alkyl group that may be substituted;
   R⁸ and R⁹ each independently represent a hydrogen atom, or a linear, branched, or cyclic alkyl group that may be substituted, or R⁸ and R⁹ may be bonded to form a ring, or R³ and R⁸ may be bonded to form a ring, and/or R⁴ and R⁹ may be bonded to form a ring;
   X represents a linear or branched C1 to C3 alkylene group;
   R¹⁰ and R¹¹ each independently represent a hydrogen atom, a hydroxyl group (OH), an alkoxy group, or a linear, branched, or cyclic alkyl group that may be substituted, or R¹⁰ and R¹¹ may be bonded to form a ring;
   R¹² represents a hydrogen atom, a linear, branched, or cyclic alkyl group that may be substituted, a linear or branched alkenyl group that may be substituted, a linear or branched alkynyl group that may be substituted, an aryl group that may be substituted, or a heterocyclic group that may be substituted, or R¹² may be bonded to R¹⁰ or R¹¹ to form a ring; and
   L represents an amide bond, an ester bond, or -(linear or branched alkylene-O-)ₙ- where "n" represents an integer of from 1 to 10, or L represents a single bond.
[2] The lyophilized preparation according to the above-mentioned item [1] may further include a solubilizing agent.
[3] In the lyophilized preparation according to the above-mentioned item [2], the solubilizing agent may contain a polyoxyethylene sorbitan fatty acid ester and/or polyoxyethylene polyoxypropylene glycol.
[4] In the lyophilized preparation according to the above-mentioned item [2] or [3], the solubilizing agent may be at least one kind selected from polysorbate 20, polysorbate 40, and polysorbate 80.
[5] In the lyophilized preparation according to the above-mentioned item [2] or [3], a content of the solubilizing agent may be from 3 parts by weight to 300 parts by weight with respect to 1 part by weight of the compound or the salt thereof.
[6] The lyophilized preparation according to any one of the above-mentioned items [1] to [5] may further include an antioxidant.
[7] In the lyophilized preparation according to the above-mentioned item [6], the antioxidant may contain methionine.
[8] In the lyophilized preparation according to the above-mentioned item [6] or [7], a content of the antioxidant may be from 0.01 part by weight to 100 parts by weight with respect to 1 part by weight of the compound or the salt thereof.
[9] The lyophilized preparation according to any one of the above-mentioned items [1] to [8] may further include at least one kind of additive selected from a pH regulator, an excipient, and a tonicity-adjusting agent.
[10] The lyophilized preparation according to any one of the above-mentioned items [1] to [9] may be a glaucoma diagnostic agent or a cancer diagnostic agent.
[11] According to another aspect of the present invention, there is provided a lyophilization solution, including: a compound represented by the general formula (I) or a salt thereof; a solubilizing agent; and water.
[12] In the lyophilization solution according to the above-mentioned item [11], the solubilizing agent may contain a polyoxyethylene sorbitan fatty acid ester and/or polyoxyethylene polyoxypropylene glycol.
[13] In the lyophilization solution according to the above-mentioned item [11] or [12], the solubilizing agent may be at least one kind selected from polysorbate 20, polysorbate 40, and polysorbate 80.
[14] The lyophilization solution according to any one of the above-mentioned items [11] to [13] may further include an antioxidant.
[15] In the lyophilization solution according to the above-mentioned item [14], the antioxidant may contain methionine.
[16] In the lyophilization solution according to any one of the above-mentioned items [11] to [15], a concentration of the compound or the salt thereof may be 6 µM or more.
[17] The lyophilization solution according to any one of the above-mentioned items [11] to [16] may further include at least one kind of additive selected from a pH regulator, an excipient, and a tonicity-adjusting agent.
[18] According to another aspect of the present invention, there is provided a method of producing the lyophilization solution of any one of the above-mentioned items [11] to [17], including: mixing the compound or the salt thereof and the solubilizing agent or an aqueous solution thereof to prepare a concentrated solution; and mixing the concentrated solution and a diluent.
[19] In the method of producing the lyophilization solution according to the above-mentioned item [18], a concentration of the solubilizing agent in the concentrated solution may be 5 wt% or more.
[20] In the method of producing the lyophilization solution according to the above-mentioned item [18] or [19], the concentrated solution and/or the diluent may contain an antioxidant.
[21] The method of producing the lyophilization solution according to any one of the above-mentioned items [18] to [20] may further include subjecting the solubilizing agent or the aqueous solution thereof, the concentrated solution, and/or the diluent to deoxygenation treatment.
[22] According to another aspect of the present invention, there is provided a method of producing a lyophilized preparation, including subjecting the lyophilization solution of any one of the above-mentioned items [11] to [17] to lyophilization treatment.
[23] According to another aspect of the present invention, there is provided a liquid preparation, which is obtained by reconstituting the lyophilized preparation of any one of the above-mentioned items [1] to [10].

### Advantageous Effects of Invention

According to the embodiment of the present invention, the above-mentioned probe is turned into the lyophilized preparation, and hence its water solubility can be improved, and the content and purity of the probe can be stabilized. The above-mentioned probe is preferably subjected to lyophilization together with the solubilizing agent.

### Brief Description of Drawings

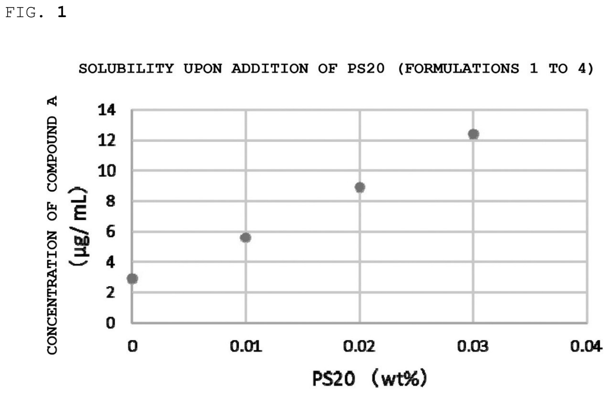

FIG. 1 is a graph showing the results of the dissolution test of Compound A.

### Description of Embodiments

Preferred embodiments of the present invention are described below, but the present invention is not limited to these embodiments. The expression "from ··· to ···" representing a numerical range in this description includes the numerical values of its upper limit and lower limit.

### A. Lyophilized Preparation

A lyophilized preparation according to an embodiment of the present invention includes a compound represented by the general formula (I) (hereinafter sometimes referred to as "compound (I)") or a salt thereof. Although the compound (I) in a powder state or the salt thereof has low water solubility, the lyophilized preparation including the compound (I) or the salt thereof can be excellent in resolubility in water or physiological saline. In addition, in the case where the compound (I) or the salt thereof is turned into the lyophilized preparation, the stability of its purity can be improved because the production of an oxidized form or the like is suppressed as compared to that in the case of a liquid preparation. The lyophilized preparation according to the embodiment of the present invention preferably further includes a solubilizing agent.

### A-1. Compound represented by Formula (I)

In the formula (I), R¹ represents a substituent bonded to a benzene ring. "p" represents an integer of from 0 to 4, and may represent from 0 to 3, from 0 to 2, 0 or 1, 1, or 0. The substituent represented by R¹ may be, for example, a linear or branched alkyl group that may be substituted, a linear or branched alkoxy group that may be substituted, a halogen atom, an amino group that may be substituted, a substituted silyl group, or an acyl group, but is not limited thereto. When "p" represents 2 or more, the plurality of R¹s may be identical to or different from each other.

When R⁸ and R⁹ are bonded to form a ring, when R³ and R⁸ are bonded to form a ring, when R⁴ and R⁹ are bonded to form a ring, or when R¹⁰ and R¹¹ are bonded to form a ring, the ring may contain a heteroatom (e.g., an oxygen atom, a nitrogen atom, or a sulfur atom), and may be, for example, a cycloalkyl group or a cyclic ether. When R¹² and R¹⁰ or R¹¹ are bonded to form a ring, the ring may be a cyclic ether. In addition, those rings may each be from 3-membered to 14-membered, from 3-membered to 10-membered, from 3-membered to 8-membered, or from 3-membered to 6-membered.

Throughout this description, examples of a substituent in the alkyl group or the alkyl moiety of the other group may include a halogen atom, a hydroxyl group, an amino group, an alkylamino group, a dialkylamino group, a thiol group, an alkylthiol group, a sulfonyl group, an alkylsulfonyl group, an alkoxy group, a cyclic ether, a carboxyl group, an alkylcarbonyl group, an alkoxycarbonyl group, an alkoxycarbonylamino group, an alkylcarbonyloxy group, an alkylaminocarbonyl group, an alkylcarbonylamino group, a carbonylamino group, and a hydrazinyl group. When the alkyl group is substituted, the number of the substituents may be set to, for example, from 1 to 6, from 1 to 5, from 1 to 4, from 1 to 3, 1 or 2, 1, 2, or 3.

A substituent in the amino group may be a linear or branched alkyl group that may be substituted. When the amino group is substituted, the number of the substituents may be set to 1 or 2, 1, or 2.

The substituent of the substituted silyl group may be a linear or branched alkyl group that may be substituted, or a phenyl group that may be substituted. The number of the substituents of the silyl group may be set to, for example, from 1 to 4, from 1 to 3, 1 or 2, 1, 2, 3, or 4.

In the general formula (I), the amino acid may be a naturally-occurring amino acid or a derivative thereof as long as the amino acid is cleaved by calpain. The amino acid may be, for example, a stereoisomer of a naturally-occurring amino acid. The amino acid may be, for example, Met, Ser, Ala, Thr, Val, Tyr, Leu, Asn, Ile, Gln, Pro, Asp, Phe, Glu, Trp, Lys, Cys, Arg, Gly, His (these amino acids are represented according to three letter codes for amino acids), norleucine (Nle), or methionine sulfoxide (MetO).

Herein, the term "alkyl group" or "alkyl" moiety in the other group means a linear, branched, or cyclic saturated hydrocarbon group. The group is preferably a saturated hydrocarbon group having 1 to 6 carbon atoms, and examples thereof include a methyl group, an ethyl group, a n-propyl group, an i-propyl group, a n-butyl group, a sec-butyl group, a t-butyl group, an isobutyl group, a pentyl group, an isopentyl group, a 2,3-dimethylpropyl group, a hexyl group, a cyclopropyl group, a cyclobutyl group, a cyclopentyl group, and a cyclohexyl group. The group is more preferably a C1-5 alkyl group, and examples thereof include a methyl group, an ethyl group, a n-propyl group, an i-propyl group, a n-butyl group, a sec-butyl group, a t-butyl group, an isobutyl group, a pentyl group, an isopentyl group, or a 2,3-dimethylpropyl group. The group is still more preferably a C1-3 alkyl group, and examples thereof include a methyl group, an ethyl group, a n-propyl group, and an i-propyl group. The group is most preferably a methyl group or an ethyl group. In addition, the term "alkylene group" refers to a divalent group obtained by removing one hydrogen atom from the above-mentioned alkyl group.

The term "alkoxy group" refers to a group to be bonded to the above-mentioned alkyl group via an oxygen atom ((alkyl group)-O- group), and the alkyl group moiety is as defined in the foregoing. For example, the number of carbon atoms of the alkyl group moiety in the alkoxy group may be from 1 to 6. Examples of the alkoxy group include a methoxy group, an ethoxy group, a 1-propyloxy group, a 2-propyloxy group, a 2-methyl-1-propyloxy group, a 2-methyl-2-propyloxy group, a 2,2-dimethyl-1-propyloxy group, a 1-butyloxy group, a 2-butyloxy group, a 2-methyl-1-butyloxy group, a 3-methyl-1-butyloxy group, a 2-methyl-2-butyloxy group, a 3-methyl-2-butyloxy group, a 1-pentyloxy group, a 2-pentyloxy group, a 3-pentyloxy group, a 2-methyl-1-pentyloxy group, a 3-methyl-1-pentyloxy group, a 2-methyl-2-pentyloxy group, a 3-methyl-2-pentyloxy group, a 1-hexyloxy group, a 2-hexyloxy group, and a 3-hexyloxy group. The C1-6 alkoxy group is preferably a C1-5 alkoxy group, more preferably a methoxy group, an ethoxy group, a n-propyloxy group, an i-propyloxy group, a n-butyloxy group, a sec-butyloxy group, a t-butyloxy group, an isobutyloxy group, a pentyloxy group, an isopentyloxy group, and a 2,3-dimethylpropyloxy group.

The term "alkenyl group" refers to a monovalent group formed by removing one hydrogen atom from any appropriate carbon atom in a linear, branched, or cyclic unsaturated hydrocarbon having one or more carbon-carbon double bonds, and may have, for example, 2 to 10, 2 to 6, or 2 to 4 carbon atoms. Examples of the C2-10 alkenyl group include a vinyl group, a propenyl group, an isopropenyl group, a 1-butenyl group, a 2-butenyl group, a 3-butenyl group, a 1-pentenyl group, a 2-pentenyl group, a 3-pentenyl group, a 4-pentenyl group, a 1-methyl-1-butenyl group, a 1-methyl-2-butenyl group, a 1-methyl-3-butenyl group, a 1-methylidenebutyl group, a 2-methyl-1-butenyl group, a 2-methyl-2-butenyl group, a 2-methyl-3-butenyl group, a 2-methylidenebutyl group, a 3-methyl-1-butenyl group, a 3-methyl-2-butenyl group, a 3-methyl-3-butenyl group, a 1-ethyl-1-propenyl group, a 1-ethyl-2-propenyl group, a 1-hexenyl group, a 2-hexenyl group, a 3-hexenyl group, a 4-hexenyl group, a 5-hexenyl group, a 1-methyl-1-pentenyl group, a 1-methyl-2-pentenyl group, a 1-methyl-3-pentenyl group, a 1-methyl-4-pentenyl group, a 1-methylidenepentyl group, a 2-methyl-1-pentenyl group, a 2-methyl-2-pentenyl group, a 2-methyl-3-pentenyl group, a 2-methyl-4-pentenyl group, a 2-methylidenepentyl group, a 3-methyl-1-pentenyl group, a 3-methyl-2-pentenyl group, a 3-methyl-3-pentenyl group, a 3-methyl-4-pentenyl group, a 3-methylidenepentyl group, a 4-methyl-1-pentenyl group, a 4-methyl-2-pentenyl group, a 4-methyl-3-pentenyl group, a 4-methyl-4-pentenyl group, a 1-heptenyl group, a 2-heptenyl group, a 3-heptenyl group, a 4-heptenyl group, a 5-heptenyl group, a 6-heptenyl group, an octenyl group, a nonenyl group, and a decenyl group.

The term "alkynyl group" refers to a monovalent group formed by removing one hydrogen atom from any appropriate carbon atom in a linear or branched unsaturated hydrocarbon having one or more carbon-carbon triple bonds, and may have, for example, 2 to 6 or 2 to 4 carbon atoms. Examples of the alkynyl group may include an ethynyl group, a 1-propynyl group, a 2-propynyl group, a 1-butynyl group, a 2-butynyl group, a 3-butynyl group, a pentynyl group, a hexynyl group, and a phenylethynyl group.

The term "acyl group" is identical in meaning to an alkanoyl group, and means a group represented by R-C(=O)-. An R moiety in the acyl group represented by the structure is, for example, a hydrogen atom, a linear or branched alkyl group that may be substituted, or an aryl group that may be substituted. The acyl group may have, for example, 2 to 7 carbon atoms, and encompasses an acetyl group, a propionyl group, a butyryl group, an isobutyryl group, a valeryl group, an isovaleryl group, a pivaloyl group, a valeryl group, an isovaleryl group, a hexanoyl group, and a heptanoyl group.

The term "aryl group" refers to a monovalent aromatic hydrocarbon group. The group may have, for example, 6 to 10 carbon atoms, and encompasses a phenyl group and a naphthyl group.

The term "heterocyclic group" means a monovalent group containing at least one of one or more kinds of heteroatoms selected from a nitrogen atom, an oxygen atom, and a sulfur atom, and the group is preferably from 5-membered to 14-membered. The heterocyclic group may be a monocyclic heterocyclic group or a fused heterocyclic group. The number of the heteroatoms incorporated into the 5-membered to 14-membered heterocyclic group may be, for example, from 1 to 5, from 1 to 4, from 1 to 3, 1 or 2, 2, or 1. For example, the following various combinations exist: a heterocyclic group containing 1 nitrogen atom; a heterocyclic group containing 2 nitrogen atoms; a heterocyclic group containing 3 nitrogen atoms; a heterocyclic group containing 1 oxygen atom; a heterocyclic group containing 2 oxygen atoms; a heterocyclic group containing 1 oxygen atom and 1 nitrogen atom; and a heterocyclic group containing 1 sulfur atom. The 5-membered to 14-membered heterocyclic group may be aromatic or nonaromatic. The monocyclic heterocyclic group is preferably a 5-membered or 6-membered ring. The fused heterocyclic group is preferably an 8-membered to 10-membered ring. Examples of the 5-membered to 14-membered heterocyclic group may include piperidyl, piperazyl, morpholyl, quinuclidyl, pyrrolidyl, azetidyl, oxetyl, azetidin-2-one-yl, aziridinyl, tropanyl, furyl, tetrahydrofuryl, thienyl, pyrrolyl, pyrrolinyl, pyrrolidinyl, dioxolanyl, oxazolyl, oxazolinyl, isoxazolyl, thiazolyl, thiazolinyl, isothiazolyl, imidazolyl, imidazolinyl, imidazolidinyl, oxazolidinyl, thiazolidinyl, pyrazolyl, pyrazolinyl, pyrazolidinyl, oxadiazolyl, furazanyl, thiadiazolyl, 1,2,3-triazolyl, 1,2,4-triazolyl, tetrazolyl, pyranyl, pyridyl, piperidinyl, pyridazinyl, pyrimidinyl, pyrazinyl, piperazinyl, dioxanyl, oxazinyl, morpholinyl, thiazinyl, triazinyl, benzofuranyl, isobenzofuranyl, dihydrobenzofuranyl, dihydroisobenzofuranyl, benzothienyl, isobenzothienyl, dihydrobenzothienyl, dihydroisobenzothienyl, tetrahydrobenzothienyl, quinolyl, isoquinolyl, quinazolinyl, phthalazinyl, pteridinyl, coumaryl, chromonyl, 1,4-benzodiazepinyl, indolyl, isoindolyl, benzimidazolyl, benzofuryl, purinyl, acridinyl, phenoxazinyl, phenothiazinyl, benzoxazolyl, benzothiazolyl, indazolyl, benzimidazolyl, benzodioxolanyl, benzodioxanylchromenyl, chromanyl, isochromanyl, chromanonyl, cinnolinyl, quinoxalinyl, indolizinyl, quinolidinyl, imidazopyridyl, naphthyridinyl, dihydrobenzoxazinyl, dihydrobenzoxazolynonyl, dihydrobenzoxazinonyl, and benzothioxanyl.

Herein, the term "halogen atom" means a fluorine atom, a chlorine atom, a bromine atom, and an iodine atom, and the halogen atom is preferably a fluorine atom, a chlorine atom, and a bromine atom, more preferably a fluorine atom or a chlorine atom. Herein, the term "carboxyl group" refers to a group represented by -C(=O)-OH.

The amino acid A is preferably Met, Thr, His, Tyr, Phe, Ser, Gln, Arg, Lys, MetO, and Nle. The amino acid B is preferably a hydrophobic amino acid, for example, Leu or Val, more preferably Leu. Preferred examples of the combination of (amino acid B)-(amino acid A) may include Leu-Met, Leu-Nle, Leu-Phe, Leu-Tyr, Leu-Thr, Leu-Arg, Leu-Lys, Leu-His, Val-Tyr, Val-Met, and Leu-Ser.

R¹ preferably represents a linear or branched alkyl group. In addition, "p" represents preferably 0 or 1, more preferably 0.

R² preferably represents a hydrogen atom.

R³ preferably represents a hydrogen atom.

R⁴ preferably represents a hydrogen atom.

R⁵ preferably represents a hydrogen atom.

R⁶ preferably represents a hydrogen atom.

R⁷ preferably represents a hydrogen atom.

R⁸ preferably represents a hydrogen atom.

R⁹ preferably represents a hydrogen atom.

R¹⁰ preferably represents a hydrogen atom, an alkyl group, or an alkoxy group, more preferably a hydrogen atom, a methyl group, or a methoxy group.

### R¹¹ preferably represents a hydrogen atom, an alkyl group, or an alkoxy group, more preferably a hydrogen atom, a methyl group, or a methoxy group.

Alternatively, R¹⁰ and R¹¹ are bonded to each other to form preferably a cycloalkyl group, more preferably a C3-C5 cycloalkyl group.

R¹² represents preferably a hydrogen atom, or a linear, branched, or cyclic alkyl group that may be substituted, more preferably a hydrogen atom, or a linear, branched, or cyclic alkyl group having 1 to 6 carbon atoms that may be substituted with one or more groups selected from a hydroxyl group, a carboxyl group, a cycloether, an alkoxy group (e.g., a methoxy group or an ethoxy group), a sulfonyl group, a halogen atom, an amino group, a methylamino group, and a dimethylamino group, still more preferably a hydrogen atom, or a linear or branched alkyl group having 1 to 4 carbon atoms (e.g., a methyl group, an ethyl group, a propyl group, an isopropyl group, or a t-butyl group) that may be substituted with one or more groups selected from a hydroxyl group, a carboxyl group, a cycloether, an alkoxy group (e.g., a methoxy group or an ethoxy group), a sulfonyl group, a halogen atom, an amino group, a methylamino group, and a dimethylamino group. Alternatively, R¹² is preferably bonded to R¹⁰ or R¹¹ to form a 3-membered to 8-membered heterocyclic group having 1 or 2 oxygen atoms (e.g., dioxolanyl or dioxanyl).

L preferably represents -(linear or branched alkylene-O-)ₙ- (where "n" represents an integer of from 1 to 10), or a single bond. The linear or branched alkylene group may be, for example, an alkylene group having 1 to 4 carbon atoms, preferably 2 or 3 carbon atoms, more preferably 2 carbon atoms.

For example, in the formula (I), a group represented by -O-L-R¹² may be a group represented by the following formula (II) : 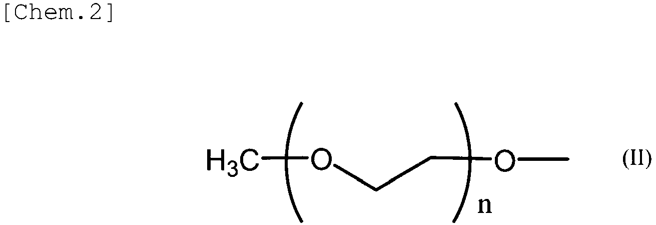 where "n" represents an integer of from 0 to 10.

In addition, for example, in the formula (I), a group represented by -O-L-R¹² may be a hydroxyl group or a group selected from the following.

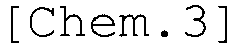

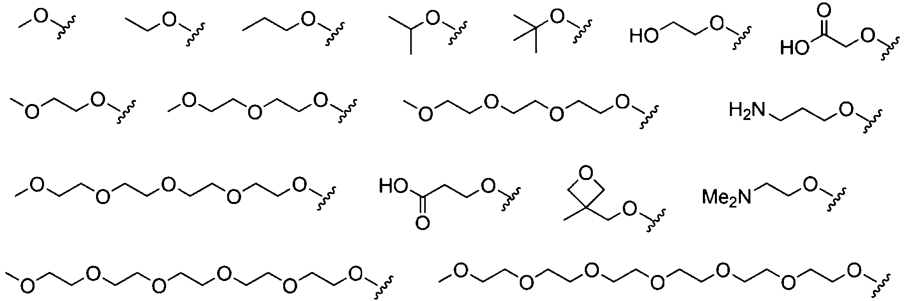

In addition, for example, in the formula (I), a partial structure represented by 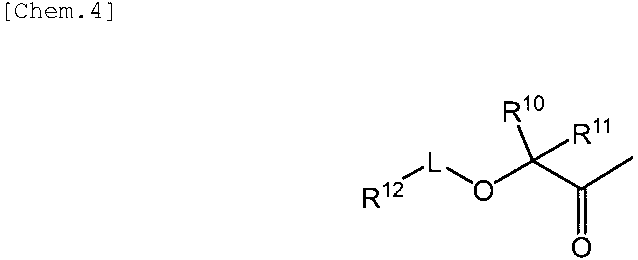 may be a structure represented by the following formula (where "m" represents an integer of from 1 to 3, and "q" represents 0 or 1) .

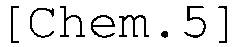

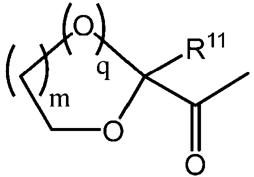

The compound (I) may be preferably, for example, a compound represented by the formula (III). In addition, a compound obtained by appropriately changing the combination of amino acids in the formula (III) may be given as a preferred example of the compound (I).

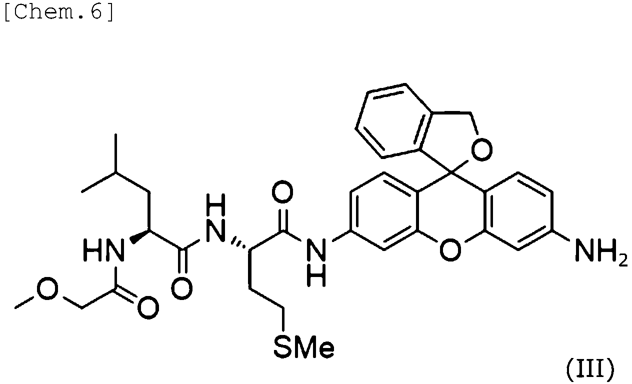

The salt of the compound (I) only needs to be a pharmaceutically acceptable salt, and examples thereof may include a base addition salt, an acid addition salt, and an amino acid salt. Examples of the base addition salt may include: metal salts, such as a lithium salt, a sodium salt, a potassium salt, a calcium salt, and a magnesium salt; organic amine salts, such as an ammonium salt, a methylamine salt, a dimethylamine salt, a dicyclohexylamine salt, a tris(hydroxymethyl)aminomethane salt, an N,N-bis(hydroxyethyl)piperazine salt, a 2-amino-2-methyl-1-propanol salt, an ethanolamine salt, an N-methylglucamine salt, an L-glucamine salt, a triethylamine salt, a piperidine salt, and a morpholine salt; and salts with basic amino acids, such as lysine, δ-hydroxylysine, and arginine. Examples of the acid addition salt may include: mineral acid salts, such as a hydrochloric acid salt, a hydrobromic acid salt, a sulfuric acid salt, a nitric acid salt, and a phosphoric acid salt; and organic acid salts, such as a methanesulfonic acid salt, a benzenesulfonic acid salt, a para-toluenesulfonic acid salt, a citric acid salt, an oxalic acid salt, an acetic acid salt, a propionic acid salt, a tartaric acid salt, a fumaric acid salt, a maleic acid salt, a malic acid salt, a succinic acid salt, a benzoic acid salt, a mandelic acid salt, a cinnamic acid salt, a lactic acid salt, a glycolic acid salt, a glucuronic acid salt, an ascorbic acid salt, a nicotinic acid salt, a salicylic acid salt, and a trifluoroacetic acid salt. The amino acid salt may be, for example, a glycine salt. Of course, the salt of the compound (I) is not limited thereto.

The compound (I) may have one or two or more asymmetric carbon atoms in accordance with the kind of a substituent thereof, and hence a stereoisomer, such as an optical isomer or a diastereoisomer, may exist. A stereoisomer in a pure form, any appropriate mixture of stereoisomers, a racemic form, and the like are each included in the scope of the present invention. In addition, although the compound represented by the general formula (I) or the salt thereof may exist as a hydrate or a solvate, these substances are each included in the scope of the present invention. Although the kind of a solvent that forms the solvate is not particularly limited, examples thereof may include solvents, such as ethanol, acetone, and isopropanol. The reference "compound (I)" as used herein encompasses any appropriate mixture of the isomers of the compound (I) or a specific stereoisomer thereof, a pharmacologically acceptable salt, hydrate, and solvate of the compound (I), and a hydrate or solvate of a pharmacologically acceptable salt of the compound (I) even when no such compound is clearly described except for the case where such compound is obviously unsuitable.

The content of the compound (I) in the lyophilized preparation only needs to be such an amount that a liquid preparation including the compound (I) at a desired concentration is obtained at the time of the reconstitution of the preparation. The content may be appropriately set by adjusting the addition amount of the solubilizing agent, an excipient, or the like.

### A-2. Solubilizing Agent

The solubilizing agent can contribute to an improvement in solubility of the compound (I). For example, a surfactant may be used as the solubilizing agent. Specifically, the solubilizing agent may be an anionic surfactant, a cationic surfactant, or a nonionic surfactant, and is preferably a nonionic surfactant.

The HLB of the solubilizing agent may be, for example, 9 or more, preferably 13 or more, more preferably 16 or more, and may be, for example, 19 or less, or for example, 18 or less. When the solubilizing agent having the above-mentioned HLB is used, an improving effect on the solubility of the compound (I) can be more suitably obtained.

Specific examples of the solubilizing agent include polyoxyethylene sorbitan fatty acid esters, specifically, polysorbate 20 (HLB: 16.7), polysorbate 21 (HLB: 13.3), polysorbate 40 (HLB: 15.6), polysorbate 60 (HLB: 14.9), polysorbate 61 (HLB: 9.6), polysorbate 65 (HLB: 10.5), polysorbate 80 (HLB: 15.0), polysorbate 81 (HLB: 10.0), and polysorbate 85 (HLB: 11.0). Of those, polysorbate 20, polysorbate 40, and polysorbate 80 are preferred, and polysorbate 20 and polysorbate 80 are more preferred. Another preferred example thereof may be polyoxyethylene polyoxypropylene glycol. The solubilizing agents may be used alone or in combination thereof.

The content of the solubilizing agent in the lyophilized preparation is, for example, from 3 parts by weight to 300 parts by weight, preferably from 10 parts by weight to 100 parts by weight, more preferably from 20 parts by weight to 50 parts by weight with respect to 1 part by weight of the compound (I). When two or more kinds of solubilizing agents are used in combination, the above-mentioned content is a total content thereof. The same applies to the content of any other component.

### A-3. Additive

The lyophilized preparation may further include any appropriate pharmaceutically acceptable additive in accordance with purposes. Examples of such additive include an antioxidant, a pH regulator, an excipient, and a tonicity-adjusting agent.

The antioxidant is not limited as long as the effects of the present invention are obtained, and preferred examples thereof include methionine, ascorbic acid, tocopherol, cysteine, thioglycerin. Of those, methionine and cysteine are preferred. The antioxidants may be used alone or in combination thereof. When the lyophilized preparation includes the antioxidant, the content of the antioxidant in the lyophilized preparation is, for example, from 0.01 part by weight to 100 parts by weight, preferably from 0.1 part by weight to 50 parts by weight, more preferably from 0.25 part by weight to 25 parts by weight with respect to 1 part by weight of the compound (I).

The pH regulator is not limited as long as the effects of the present invention are obtained, and preferred examples thereof include hydrochloric acid, sodium hydroxide, phosphoric acid, phosphoric acid salts (e.g., Na₃PO₄, Na₂HPO₄, NaH₂PO₄, and KH₂PO₄), carbonic acid, carbonic acid salts (e.g., Na₂CO₃ and NaHCO₃), acetic acid, acetic acid salts (e.g., CH₃COONa), citric acid, citric acid salts (e.g., trisodium citrate), and HEPES. The pH regulators may be used alone or in combination thereof. The pH regulator may form a buffering agent. The pH regulator may be incorporated into the lyophilized preparation so that the pH of the lyophilized preparation may be, for example, from 6 to 8 at the time of the dissolution thereof in physiological saline or water for injection.

The excipient is not limited as long as the effects of the present invention are obtained, and preferred examples thereof include: monosaccharides such as glucose; disaccharides, such as sucrose, trehalose, maltose, and lactose; and sugar alcohols, such as mannitol and sorbitol. Of those, trehalose and sucrose are preferred. The excipients may be used alone or in combination thereof. When the lyophilized preparation includes the excipient, the content of the excipient in the lyophilized preparation is, for example, from 1 part by weight to 25,000 parts by weight, preferably from 10 parts by weight to 10,000 parts by weight, more preferably from 100 parts by weight to 5,000 parts by weight with respect to 1 part by weight of the compound (I).

The tonicity-adjusting agent is not limited as long as the effects of the present invention are obtained, and preferred examples thereof include sodium chloride and potassium chloride. The tonicity-adjusting agents may be used alone or in combination thereof. The tonicity-adjusting agent may be added to the lyophilized preparation so that when the lyophilized preparation is dissolved in a dissolving liquid to prepare an administration liquid having a predetermined concentration, the ratio of the osmotic pressure of the administration liquid to that of physiological saline may be from 0.8 to 1.2.

### B. Lyophilization Solution

According to another aspect of the present invention, there is provided a lyophilization solution including the compound (I), a solubilizing agent, and water. The lyophilization solution according to the embodiment of the present invention preferably further includes an antioxidant, and may include any appropriate other additive as required. The solubilizing agent, the antioxidant, and the other additive are as described in the section A. Preferred examples of the water include water for injection, purified water, and sterile purified water. The lyophilized preparation described in the section A may be suitably obtained by lyophilizing the lyophilization solution according to the embodiment of the present invention.

The concentration of the compound (I) in the lyophilization solution is, for example, 6 µM or more, preferably 60 µM or more, more preferably 120 µM or more, still more preferably 240 µM or more, and is, for example, 10 mM or less, or for example, 1 mM or less.

The concentration of the solubilizing agent in the lyophilization solution is, for example, from 0.05 mg/mL to 50 mg/mL, preferably from 1 mg/mL to 20 mg/mL, more preferably from 2 mg/mL to 10 mg/mL. In the lyophilization solution including the solubilizing agent at the above-mentioned concentration, the compound (I) can be stably dissolved at a high concentration. In addition, a lyophilized preparation produced from the lyophilization solution including the solubilizing agent at the above-mentioned concentration can be easily dissolved in a dissolving liquid.

When the lyophilization solution includes the antioxidant, the concentration of the antioxidant in the lyophilization solution is, for example, from 0.001 mg/mL to 10 mg/mL, preferably from 0.01 mg/mL to 5 mg/mL, more preferably from 0.02 mg/mL to 1 mg/mL. In the lyophilization solution including the antioxidant at the above-mentioned concentration, a reduction in purity can be suppressed because the modification (e.g., oxidation) of the compound (I) can be suppressed.

When the lyophilization solution includes a pH regulator, the concentration of the pH regulator in the lyophilization solution is not limited as long as the effects of the present invention are obtained. When the pH regulator is a phosphate (e.g., when the pH regulator is a phosphate buffering agent), its concentration is, for example, from 1 mM to 500 mM, preferably from 2 mM to 300 mM, more preferably from 3 mM to 150 mM.

When the lyophilization solution includes an excipient, the concentration of the excipient in the lyophilization solution is, for example, from 1 mg/mL to 400 mg/mL, preferably from 5 mg/mL to 200 mg/mL, more preferably from 10 mg/mL to 100 mg/mL. According to the lyophilization solution including the excipient at the above-mentioned concentration, a lyophilized preparation excellent in handleability can be suitably produced.

### C. Method of producing Lyophilization Solution

According to another aspect of the present invention, there is provided a method of producing a lyophilization solution. The method of producing a lyophilization solution according to the embodiment of the present invention includes:
(step I) mixing the compound (I) and a solubilizing agent or an aqueous solution thereof to prepare a concentrated solution; and
(step II) mixing the above-mentioned concentrated solution and a diluent.

According to the above-mentioned production method, when the compound (I) is dissolved in the presence of the solubilizing agent, a concentrated solution in which the compound (I) is dissolved at a high concentration can be obtained, and a lyophilization solution including the compound (I) at a desired concentration can be obtained by diluting the concentrated solution. Accordingly, according to the above-mentioned production method, the lyophilization solution described in the section B can be suitably produced.

The above-mentioned production method may further include subjecting the solubilizing agent or the aqueous solution thereof described above, the above-mentioned concentrated solution, and/or the above-mentioned diluent to deoxygenation treatment.

### C-1. Step I

In the step I, the compound (I) and the solubilizing agent or the aqueous solution thereof are mixed to prepare the concentrated solution. The compound (I) and the solubilizing agent may be directly mixed (without addition of water), or the compound (I) and the aqueous solution of the solubilizing agent may be mixed. The solubilizing agent or the aqueous solution of the solubilizing agent may contain any one of the various additives. For example, the aqueous solution of the solubilizing agent may be an aqueous solution obtained by dissolving the solubilizing agent in water, a buffer, or physiological saline.

The concentration of the solubilizing agent in the aqueous solution of the solubilizing agent at the time of the mixing with the compound (I) is, for example, 5 wt% or more, preferably 10 wt% or more, more preferably 20 wt% or more. When the compound (I) is dissolved in the presence of such concentration of the solubilizing agent, a concentrated solution in which the compound (I) is dissolved at a high concentration can be obtained. The concentration of the solubilizing agent in the concentrated solution is, for example, 5 wt% or more, preferably 10 wt% or more, more preferably 20 wt% or more, and is, for example, 99.7 wt% or less, or for example, 98 wt% or less.

The concentration of the compound (I) in the concentrated solution is, for example, from 0.1 mg/g to 300 mg/g, preferably from 1 mg/g to 150 mg/g, more preferably from 2 mg/g to 50 mg/g.

The concentrated solution may appropriately contain an additive (e.g., an antioxidant, an excipient, a pH regulator, or a tonicity-adjusting agent) in accordance with the target composition of the lyophilization solution. The kind and concentration of the additive in the concentrated solution may be appropriately set in accordance with the target composition of the lyophilization solution. The additive may be mixed with the compound (I) after having been added to the solubilizing agent or the aqueous solution thereof as described above, or may be separately added at the time of the mixing of the solubilizing agent or the aqueous solution thereof and the compound (I).

The concentrated solution preferably contains an antioxidant. The concentration of the antioxidant in the concentrated solution is, for example, from 0.1 mg/g to 300 mg/g, preferably from 0.5 mg/g to 150 mg/g, more preferably from 1 mg/g to 50 mg/g. When the concentrated solution contains the antioxidant, the production of an oxidized form of the compound (I) can be suppressed.

The compound (I) and the solubilizing agent or the aqueous solution thereof may be mixed by, for example, stirring, shaking, or irradiation with an ultrasonic wave. A mixing time may be set to, for example, from 30 minutes to 120 hours, preferably from 2 hours to 48 hours. A mixing temperature may be set to, for example, from 0°C to 50°C, preferably from 10°C to 30°C.

### C-2. Step II

In the step II, the above-mentioned concentrated solution and the diluent are mixed. The diluent contains water, and may further contain an additive (e.g., an antioxidant, an excipient, a pH regulator, or a tonicity-adjusting agent) as required. The diluent containing the antioxidant can suppress the production of an oxidized form of the compound (I). The kind and concentration of the additive in the diluent may be appropriately set in accordance with the target composition of the lyophilization solution. For example, the diluent may be water, a buffer, or physiological saline. Alternatively, the additive may be separately added at the time of the mixing of the concentrated solution and the diluent.

The concentrated solution and the diluent may be mixed by, for example, stirring, shaking, or irradiation with an ultrasonic wave. A mixing time may be set to, for example, from 10 seconds to 48 hours, preferably from 1 minute to 2 hours. A mixing temperature may be set to, for example, from 0°C to 50°C, preferably from 10°C to 30°C.

The step II provides the lyophilization solution. The concentrations of the respective components (e.g., the compound (I), the solubilizing agent, the antioxidant, the excipient, the pH regulator, and the tonicity-adjusting agent) in the lyophilization solution are as described in the section B.

In the above-mentioned method of producing a lyophilization solution, the solubilizing agent or the aqueous solution thereof, the concentrated solution, and/or the diluent may be subjected to deoxygenation treatment. Thus, the production of an oxidized form of the compound (I) in the lyophilization solution or a liquid preparation reconstituted from the lyophilization solution can be suppressed. Examples of the deoxygenation treatment include nitrogen bubbling treatment and degassing treatment. Conditions for the treatment are not particularly limited, and may be set to any appropriate conditions under which the effects of the present invention can be obtained.

### D. Method of producing Lyophilized Preparation

The lyophilized preparation described in the section A may be produced by subjecting the lyophilization solution described in the section B to lyophilization treatment. For example, the lyophilization solution described in the section B is loaded into a vessel such as a vial, and is preliminarily frozen. A pressure in the vessel is reduced, and the frozen product is primarily dried (sublimation drying) and then secondarily dried (removal of bonding water). The moisture content of the lyophilized preparation to be obtained is, for example, 8 wt% or less, preferably 5 wt% or less, more preferably 3 wt% or less. Conditions for the lyophilization treatment may be appropriately set in accordance with, for example, the composition and amount of the lyophilization solution. The lyophilization treatment may be performed with a commercial lyophilizer. The produced lyophilized preparation may be supplied after having been sealed in a vessel such as a vial. The sealing is performed by, for example, vacuum sealing or nitrogen purging sealing.

### E. Usage Method and Application of Lyophilized Preparation

An administration liquid may be obtained by reconstituting the lyophilized preparation described in the section A. The term "reconstitution" means that the lyophilized preparation is dissolved in a dissolving liquid to prepare a liquid preparation again. As described above, the above-mentioned lyophilized preparation is excellent in water solubility. Accordingly, an administration liquid containing the compound (I) at a practically sufficient concentration can be easily prepared before use.

The liquid preparation may be, for example, an injection, a spray, an eye drop, or an external liquid preparation. In one embodiment, the liquid preparation is a vitreous injection, and may be injected into the vitreous body of a patient through injection.

A pharmaceutically acceptable solvent may be appropriately used as the dissolving liquid. Specific examples of the dissolving liquid include water (e.g., water for injection), physiological saline, and various buffers (e.g., a phosphate buffer, phosphate-buffered saline, and a citrate buffer). The pH of the liquid preparation is, for example, from 6 to 8.

The concentration of the compound (I) in the liquid preparation may be appropriately set in accordance with purposes, applications, and the like. The above-mentioned concentration may be, for example, from 3 µg/mL to 60 µg/mL. In addition, the above-mentioned concentration may be, for example, from 5 µM to 100 µM. The concentration of the solubilizing agent in the liquid preparation may be, for example, from 0.01 wt% to 1.5 wt%, preferably from 0.02 wt% to 1 wt%.

The purity of the compound (I) in the liquid preparation is, for example, 90% or more, preferably 92% or more, more preferably 95% or more. When the compound (I) is turned into the lyophilized preparation, for example, the production of an oxidized form thereof during storage can be suppressed. As a result, a liquid preparation including the compound (I) at high purity can be easily and quickly prepared in accordance with the time of its administration.

The lyophilized preparation and the liquid preparation described above may each be used in, for example, the diagnosis of a calpain activity-associated disease, or the judgment of the indication or therapeutic effect of a calpain inhibitor on the disease because the above-mentioned compound (I) can perform the fluorescence imaging of calpain activity.

The calpain activity-associated disease may be such a disease that the expression of a calpain or the activity thereof contributes to its onset or exacerbation. Examples of the calpain activity-associated disease may include: eye diseases, such as glaucoma including normal-tension glaucoma, autosomal dominant neovascular inflammatory vitreoretinopathy, retinitis pigmentosa, age-related macular degeneration, a retinal neuropathy or a retinal vascular occlusive disease associated with diabetes, retinal ischemia, cataract, normal-tension glaucoma, and other retinal diseases or retinal neuropathies; muscle diseases such as muscular dystrophy; diabetes; diabetes; inflammatory diseases or autoimmune diseases, such as acute esophagitis, multiple sclerosis, an idiopathic inflammatory muscle disease, and autoimmune encephalitis; neurological diseases, such as spinal cord injury, Parkinson's disease, a neurodegenerative disease, lissencephaly, Alzheimer's disease, and spinocerebellar degeneration; cardiovascular diseases, such as cardiac ischemia-reperfusion injury, a chronic heart disease, an abdominal aortic aneurysm, and atherosclerosis; cancers, such as a melanoma, breast cancer, and a brain tumor; an aging syndrome and progeria; infectious diseases caused by parasites or pathogenic microorganisms, such as malaria, trypanosomiasis, African trypanosomiasis, schistosomiasis, leishmaniasis, and sickle cell disease; a traumatic brain injury; Machado-Joseph disease; preeclampsia; and pulmonary fibrosis. The application of the lyophilized preparation to an eye disease including glaucoma and a cancer such as a brain tumor out of those diseases has been preferably investigated.

### Examples

The present invention is specifically described below by way of Examples, but the present invention is by no means limited to these Examples. Each operation was performed at room temperature (about 25°C) unless otherwise specified. In addition, HPLC measurement was performed under the following conditions.

### <HPLC Measurement Conditions>

·Column: Meteoric Core C8, 5 µm, 4.6 mm×150 mm (YMC Co., Ltd.)
·Column temperature: 25°C
·Feeding amount: 1.0 mL/min.
·Wavelength: 254 nm
·Mobile phase A: water/TFA (1,000:1), Mobile phase B: acetonitrile/TFA (1,000:1)
·Feeding of mobile phase

**Table 1**

| Time after injection (min) | Mobile phase A (vol%) | Mobile phase B (vol%) |
|---|---|---|
| 0 to 10 | 85 to 30 | 15 to 70 |

### [Production Example 1: Synthesis of Compound A]

A TFA salt of Compound A represented below was synthesized in accordance with a method described in Patent Literature 1. The resultant TFA salt (14.7 mg) was dissolved in 2 mL of dichloromethane, and 1 mL of a saturated aqueous solution of sodium hydrogen carbonate was added to the solution, followed by mixing. The mixed liquid was extracted with dichloromethane, and the organic phase was dried with sodium sulfate. The organic solvent was evaporated under reduced pressure. Thus, Compound A serving as a desalted form was obtained (pale pink solid, 9.9 mg, 79%).

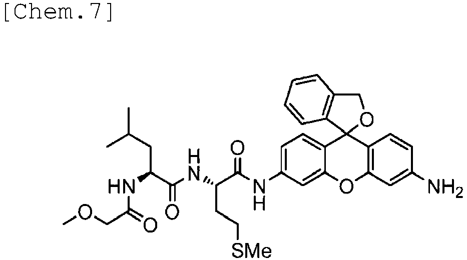

### [Test Example 1: Dissolution Test of Compound A]

1 Milligram of Compound A and 10 mL of a 6.55 mM phosphate buffer (pH: 7) containing 0 wt% to 0.03 wt% of polysorbate 20 (FUJIFILM Wako Pure Chemical Corporation, hereinafter referred to as "PS20") or an aqueous solution of NaH₂PO₄ (0.33 mg/mL) containing 0 wt% to 0.03 wt% of PS20 were loaded into a glass vial having a volume of 20 mL, and air inside the vial was purged with nitrogen. The vial was loaded into Aluminum Lamizip, and the vial was shaken overnight. The phosphate buffer was prepared by using NaH₂PO₄ and Na₂HPO₄. The mixture was filtered with a PVDF filter (Millex-GV, 0.22 µm, PVDF, 4 mm), and the solution after 10 drops of its initial flow was collected. Thus, solutions having formulations 1 to 8 were obtained. The concentrations of Compound A in the resultant solutions were calculated by the HPLC measurement of the solutions. The results are shown in the following table and FIG. 1.

**Table 2**

| Formulation | PS20 (wt%) | Buffer | conc (µg/mL) |
|---|---|---|---|
| 1 | 0 | Phosphate buffer | 2.9 |
| 2 | 0.01 | Phosphate buffer | 5.6 |
| 3 | 0.02 | Phosphate buffer | 8.9 |
| 4 | 0.03 | Phosphate buffer | 12.4 |
| 5 | 0 | NaH₂PO₄ | 3.6 |
| 6 | 0.01 | NaH₂PO₄ | 6.2 |
| 7 | 0.02 | NaH₂PO₄ | 9.9 |
| 8 | 0.03 | NaH₂PO₄ | 13.4 |

In each of the formulations 1 to 4 (phosphate buffers) and the formulations 5 to 8 (aqueous solutions of NaH₂PO₄), the dissolved concentration of Compound A increased as the concentration of PS20 increased. The use of PS20 as a solubilizing agent was effective in increasing the dissolved concentration of Compound A.

### [Test Example 2: Method of dissolving Compound A]

The solutions of Compound A were prepared by Methods A to C below, and the concentrations of Compound A in the solutions were measured by HPLC. A sample obtained as follows was used as a HPLC measurement sample (the same applies to the following HPLC measurement): each of the solutions was filtered with a PVDF filter (Millex-GV, 0.22 µm, PVDF, 4 mm), and the solution after 10 drops of its initial flow was collected. The compositions of the solutions and the measured values of the concentrations of Compound A therein are shown in the following table.

### ·Method A

The powder of Compound A was weighed in a glass vial, and was added to a 0.03 wt% or 0.3 wt% aqueous solution of PS20 containing various additives so that the concentration of Compound A was 11.4 µg/mL or 114 µg/mL, followed by stirring overnight. Thus, solutions having formulations 9 and 10 were prepared.

### ·Method B

56.3 Milligrams of Compound A was precisely weighed in a brown vial, and PS20 was added thereto so that the concentration of Compound A was 36.5 mg/g.

A stirring bar was loaded into the above-mentioned vial, and the vial was sealed, followed by stirring under light shielding overnight. Thus, Compound A was dissolved in PS20. The above-mentioned solution was weighed in a glass vial, and an aqueous solution containing various additives was added thereto so that the concentration of Compound A was 11.4 µg/mL or 114 µg/mL, followed by mixing by ultrasonic vibration for 1 minute. Thus, solutions having formulations 11 to 13 were prepared.

### ·Method C

Compound A was precisely weighed in a brown vial, and a 20 wt% aqueous solution of PS20 was added thereto so that the concentration of Compound A was 7.30 mg/g, followed by stirring overnight in the same manner as in Method B. Thus, Compound A was dissolved. The above-mentioned solution was weighed in a glass vial, and an aqueous solution containing various additives was added thereto so that the concentration of Compound A was 114 µg/mL, followed by mixing by ultrasonic vibration for 1 minute. Thus, a solution having a formulation 14 was prepared.

**Table 3**

| Formulation | Method | Dissolution time | Solution composition | | | | | | |
|---|---|---|---|---|---|---|---|---|---|
| | | | Target concentration (µg/mL) | Measured concentration (µg/mL) | PS20 (mg/mL) | NaH₂PO₄ (mg/mL) | Na₂HPO₄ (mg/mL) | Trehalose (mg/mL) | NaCl (mg/mL) |
| 9 | A | 1 Day | 11.4 | 0.72 | 0.3 | 0.33 | 0.54 | 48 | - |
| 10 | A | 1 Day | 114 | 9.23 | 3 | 3.3 | 5.4 | 100 | - |
| 11 | B | <1 Minute | 11.4 | 11.1 | 0.3 | 0.33 | 0.54 | - | 2.3 |
| 12 | B | <1 Minute | 114 | 111 | 3 | 3.3 | 5.4 | - | 23 |
| 13 | B | <1 Minute | 114 | 116 | 3 | 3.3 | 5.4 | 100 | - |
| 14 | C | <1 Minute | 114 | 111 | 3 | 3.3 | 5.4 | 100 | - |

In Test Example 1, the solubility of Compound A in the 0.03 wt% PS20 solution (phosphate buffer) was 12.4 µg/mL, but the measured concentrations of Compound A of the formulations 9 and 10 were 0.72 µg/mL and 9.23 µg/mL, respectively, and hence the concentrations of the solutions were each less than 1/10 of the target concentration, which was much less than the target concentration. In each of the solutions, the undissolved residue of the powder of Compound A was visually observed, and it was difficult to dissolve Compound A from a powder state even when a time period of 1 day was spent thereon.

Meanwhile, in each of the formulations 11 to 14, the measured concentration of Compound A was generally the target concentration. In addition, the solution of Compound A in PS20 was quickly dissolved in the phosphate buffer, and hence no undissolved residue was visually observed. Compound A was soluble in the 20 wt% aqueous solution of PS20, and hence a solution was able to be prepared by using the solution. The following was effective in preparing a solution having a prescribed concentration in a short time period: Compound A was dissolved in PS20 or the aqueous solution of PS20 in advance.

### [Test Example 3: Investigation 1 on Stability of Lyophilized Preparation of Compound A]

NaH₂PO₄ and Na₂HPO₄ were dissolved in distilled water, and the pH of the solution was appropriately adjusted with 2 M hydrochloric acid or a 2 M aqueous solution of sodium hydroxide. Thus, phosphate buffers (2.5 mM to 250 mM) each having a pH of 7.0 were prepared. Solutions having various compositions were prepared by using the resultant phosphate buffers in accordance with Method B.

Each of the solutions was loaded in an amount of 1 mL per 10-milliliter brown vial (Daiwa Special Glass Co., Ltd.), and the vial was hermetically sealed with a laminate-coated rubber stopper (Daikyo Seiko, Ltd.) and capped with an aluminum cap. Thus, liquid preparations having formulations 15 to 19 were obtained.

The respective solutions used in the formulations 15 to 19 were loaded as lyophilization solutions having formulations 20 to 24, respectively in amounts of 1 mL each per 10-milliliter brown vial (Daiwa Special Glass Co., Ltd.), and a laminate-coated rubber stopper (Daikyo Seiko, Ltd.) was mounted on the vial, followed by freezing at -30°C or less. After a pressure in each of the vials had been reduced, a temperature therein was increased to -5°C over 4 hours, and the frozen product was primarily dried for 36 hours. The temperature was increased to 20°C over 2 hours, and the dried product was secondarily dried for 2 hours. The vial was hermetically sealed with the rubber stopper while a vacuum state therein was kept, followed by capping with an aluminum cap. Thus, lyophilized preparations having the formulations 20 to 24 were obtained. The solution compositions of the respective formulations are shown in the following table. The concentrations of Compound A are values measured by HPLC measurement.

**Table 4**

| Formulation | Solution composition | | | |
|---|---|---|---|---|
| | Compound A (µg/mL) | PS20 (mg/mL) | Buffer concentration (mM) | Trehalose (mg/mL) |
| 15 (20) | 6.30 | 0.17 | 2.5 | 100 |
| 16 (21) | 60.29 | 1.67 | 25 | 100 |
| 17 (22) | 121.09 | 3.33 | 50 | 100 |
| 18 (23) | 301.89 | 8.33 | 125 | 100 |
| 19 (24) | 593.16 | 16.67 | 250 | 100 |

Each of the preparations having the formulations 15 to 24 was loaded into Aluminum Lamizip, and was stored under room-temperature and light-shielding conditions. The contents and purities of Compound A, the contents of an oxidized form serving as the main decomposition product of Compound A, and total analog contents at the time point of the preparation of each of the formulations and at the time point of the storage thereof were determined by HPLC measurement. A test solution for measuring the purity of a lyophilized preparation was prepared by adding 1 mL of distilled water to the preparation, and vertically shaking the mixture 10 times. A solution for measuring the content of Compound A of a lyophilized preparation was prepared by adding an appropriate amount of distilled water to the preparation so that the net content thereof per one vial was 1 mL. As described above, each of the solutions was filtered with a PVDF filter (Millex-GV, 0.22 µm, PVDF, 4 mm), and the solution after 10 drops of its initial flow was adopted as a test solution.

The solubility of each of the lyophilized preparations (formulations 20 to 24) in distilled water was satisfactory. Each of the preparations was able to be dissolved by adding distilled water thereto, and vertically shaking the mixture 10 times. A time period required for the dissolution was less than 1 minute.

The contents and purities of Compound A, oxidized form contents, and total analog contents at the respective time points of the respective formulations are shown in the following table. The contents (%) of Compound A were each calculated as a ratio with respect to the concentration of Compound A of a lyophilization solution on a 0th day. In addition, the purities of Compound A, the oxidized form contents, and the total analog contents were each calculated by an area normalization method based on a peak area in a HPLC chromatogram. The same applies to the following test examples.

**Table 5**

| Formulation | Content of Compound A (%) | | | | Purity (%) | | | | Oxidized form (%) | | | | Total analog (%) | | | |
|---|---|---|---|---|---|---|---|---|---|---|---|---|---|---|---|---|
| | Day 0 | Day 4 | Day 7 | Day 14 | Day 0 | Day 4 | Day 7 | Day 14 | Day 0 | Day 4 | Day 7 | Day 14 | Day 0 | Day 4 | Day 7 | Day 14 |
| 15 | 100.0 | 28.1 | 19.6 | 16.2 | 93.2 | 71.6 | 63.8 | 57.5 | 2.98 | 13.54 | 20.27 | 27.96 | 6.79 | 28.36 | 36.25 | 42.50 |
| 16 | 100.0 | 22.8 | 19.9 | 18.3 | 96.6 | 89.7 | 87.4 | 83.4 | 2.85 | 8.79 | 10.63 | 14.45 | 3.44 | 10.26 | 12.57 | 16.60 |
| 17 | 100.0 | 21.9 | 19.7 | 18.2 | 97.0 | 91.9 | 90.6 | 88.3 | 2.22 | 7.07 | 8.30 | 10.44 | 3.04 | 8.06 | 9.39 | 11.74 |
| 18 | 100.0 | 22.8 | 20.6 | 19.1 | 96.9 | 91.6 | 90.8 | 89.5 | 2.18 | 6.90 | 7.83 | 9.18 | 3.13 | 8.37 | 9.18 | 10.54 |
| 19 | 100.0 | 21.9 | 17.9 | 15.9 | 97.0 | 92.1 | 90.8 | 89.2 | 2.00 | 6.48 | 7.79 | 9.23 | 2.97 | 7.94 | 9.23 | 10.78 |
| 20 | - | - | - | - | 90.8 | - | 91.2 | 91.6 | 3.69 | - | 4.15 | 4.46 | 9.23 | - | 8.77 | 8.43 |
| 21 | - | - | - | - | 97.1 | - | 96.7 | 96.6 | 2.61 | - | 2.72 | 2.72 | 2.95 | - | 3.26 | 3.43 |
| 22 | 101.4 | - | 103.0 | 100.1 | 97.0 | - | 96.8 | 96.7 | 2.18 | - | 2.23 | 2.21 | 2.98 | - | 3.18 | 3.29 |
| 23 | - | - | - | - | 96.8 | - | 96.7 | 96.6 | 2.21 | - | 2.19 | 2.29 | 3.17 | - | 3.28 | 3.38 |
| 24 | - | - | - | - | 97.0 | - | 96.9 | 96.9 | 1.99 | - | 2.25 | 2.08 | 2.99 | - | 3.07 | 3.12 |

In each of the liquid preparations (formulations 15 to 19), both the contents and purities of Compound A at the time points of the storage (day 4, day 7, and day 14) notably reduced. In particular, an increase in content of the oxidized form serving as the main decomposition product was remarkably observed.

In each of the lyophilized preparations (formulations 20 to 24), as compared to the liquid preparations, a reduction in purity of Compound A was able to be suppressed, and the production of the oxidized form serving as a main factor for the reduction in purity was moderate. In addition, in the formulation 22, the maintenance of the content of Compound A was recognized. Further, a time period required for the redissolution of the formulation 22 in water was less than 1 minute. Accordingly, while it was difficult to dissolve the powder of Compound A in Test Example 1, the lyophilized preparation was able to be quickly dissolved.

As described above, each of the lyophilized preparations is effective in stabilizing the content and purity of Compound A, and is excellent in resolubility in water.

The structure of the oxidized form serving as the main decomposition product of Compound A was identified by HPLC/MS analysis. As a result, the oxidized form had a structure represented below.

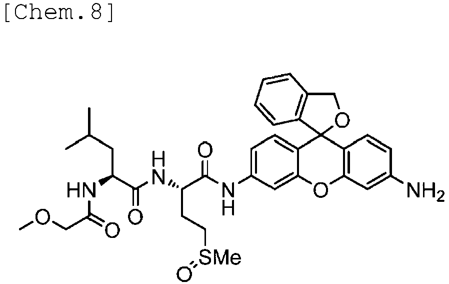

### [Test Example 4: Investigation 2 on Stability of Lyophilized Preparation of Compound A]

Lyophilized preparations having formulations 25 to 29 were produced by the same approach as that of Test Example 3. The compositions of lyophilization solutions for obtaining the respective formulations are as shown in the following table. In the table, the concentrations of Compound A are measured values.

**Table 6**

| Formulation | Composition of lyophilization solution | | | |
|---|---|---|---|---|
| | Compound A (µg/mL) | PS20 (mg/mL) | Buffer concentration (mM) | Trehalose (mg/mL) |
| 25 | 123.2 | 3.33 | 50 | 50 |
| 26 | 123.1 | 3.33 | 50 | 200 |
| 27 | 123.1 | 3.33 | 50 | 300 |
| 28 | 123.9 | 6.67 | 50 | 100 |
| 29 | 125.4 | 10.0 | 50 | 100 |

Each of the lyophilized preparations having the formulations 25 to 29 was loaded into Aluminum Lamizip, and was stored under room-temperature and light-shielding conditions. The purities of Compound A, the contents of an oxidized form serving as the main decomposition product of Compound A, and total analog contents at the time point of the preparation of each of the formulations and at the time point of the storage thereof were calculated by HPLC measurement (area normalization). A test solution for measuring the purity of a lyophilized preparation was prepared by adding 1 mL of distilled water to the preparation, and vertically shaking the mixture 10 times. The water solubility of each of the formulations except the formulation 27 was satisfactory. Specifically, the formulations 25, 26, 28, and 29 were each able to be dissolved by adding water thereto, and then vertically shaking the mixture 10 times. A time period required for the dissolution was less than 1 minute. The formulation 27 was able to be dissolved within 3 minutes by adding water thereto, and then vertically shaking the mixture 10 or more times. The purities of Compound A, oxidized form contents, and total analog contents at the respective time points of the respective formulations were as shown in the following table.

**Table 7**

| Formulation | Purity (%) | | | Oxidized form (%) | | | Total analog (%) | | |
|---|---|---|---|---|---|---|---|---|---|
| | Day 0 | Day 7 | Day 14 | Day 0 | Day 7 | Day 14 | Day 0 | Day 7 | Day 14 |
| 25 | 96.9 | 96.8 | 97.1 | 2.25 | 2.28 | 2.28 | 3.14 | 3.22 | 2.95 |
| 26 | 97.0 | 96.7 | 96.6 | 2.21 | 2.34 | 2.35 | 2.98 | 3.30 | 3.37 |
| 27 | 96.8 | 95.8 | 96.5 | 2.34 | 2.42 | 2.44 | 3.19 | 4.20 | 3.53 |
| 28 | 96.5 | 95.3 | 96.3 | 2.71 | 2.84 | 2.84 | 3.54 | 4.74 | 3.75 |
| 29 | 96.2 | 96.0 | 95.7 | 2.99 | 3.21 | 3.28 | 3.81 | 4.04 | 4.31 |

In each of the formulations 25 to 29, trehalose was incorporated as an excipient at a concentration of from 50 mg/mL to 300 mg/mL into the lyophilization solution, and the purity of Compound A and an oxidized form production amount were at the same level as those of the formulation 22.

In the formulations 25, 28, and 29, the weight ratios of PS20 to Compound A correspond to 27.0 times, 53.8 times, and 79.7 times, respectively. In each of the lyophilized preparations, the production of the oxidized form was moderate as compared to the formulations 15 to 19. Accordingly, lyophilized preparations having PS20 contents in the same range were able to be produced.

### [Test Example 5: Investigation 3 on Stability of Lyophilized Preparation of Compound A]

According to Test Example 3, a main factor for a reduction in purity of Compound A in a preparation was the decomposition of Compound A into the oxidized form. In view of the foregoing, lyophilized preparations having respective formulations were produced as described below for the purpose of suppressing the oxidized form.

PS20 and a 20 wt% aqueous solution of PS20 were subjected to nitrogen bubbling for 20 minutes. Compound A was weighed in a 3-milliliter brown glass vial, and PS20 and the 20 wt% aqueous solution of PS20 described above were each added thereto so that the concentration of Compound A was 36.5 mg/g or 7.30 mg/g, respectively. The vial was sealed, and the mixture was stirred under room temperature and light shielding overnight. Thus, Compound A was dissolved.

A phosphate buffer subjected to nitrogen bubbling for 20 minutes was added to each of the above-mentioned solutions of Compound A, and the solution was dissolved in the buffer by ultrasonic vibration for 1 minute. Thus, lyophilization solutions having compositions shown in the following table were prepared. In the table, the concentrations of Compound A are measured values.

**Table 8**

| Formulation | Composition of lyophilization solution | | | | |
|---|---|---|---|---|---|
| | Compound A (µg/mL) | PS20 (mg/mL) | NaH₂PO₄ (mg/mL) | Na₂HPO₄ (mg/mL) | Trehalose (mg/mL) |
| 30 | 103.4 | 3 | 3.3 | 5.4 | 100 |
| 31 | 111.1 | 3 | 3.3 | 5.4 | 100 |

The formulation 30 is a lyophilization solution prepared by using PS20, and the formulation 31 is a lyophilization solution prepared by using the 20 wt% aqueous solution of PS20.

Each of the lyophilization solutions was loaded in an amount of 0.5 mL per 10-milliliter brown vial (Daiwa Special Glass Co., Ltd.), and a laminate-coated rubber stopper (Daikyo Seiko, Ltd.) was mounted on the vial, followed by freezing at - 30°C or less. After a pressure in each of the vials had been reduced, a temperature therein was increased to -5°C over 4 hours, and the frozen product was primarily dried for 24 hours. The temperature was increased to 20°C over 2 hours, and the dried product was secondarily dried for 2 hours. The vial was hermetically sealed with the rubber stopper while a vacuum state therein was kept, followed by capping with an aluminum cap. Thus, lyophilized preparations were obtained (formulations 30 and 31).

Each of the preparations having the formulations 30 and 31 was loaded into Aluminum Lamizip, and was stored under room-temperature and light-shielding conditions. The purities of Compound A, the contents of an oxidized form serving as the main decomposition product of Compound A, and total analog contents at the time point of the preparation of each of the formulations and at the time point of the storage thereof were calculated by HPLC measurement (area normalization). A test solution for measuring the purity of a lyophilized preparation was prepared by adding 0.5 mL of distilled water, and vertically shaking the mixture 10 times. Each of the formulations showed satisfactory water solubility, and was able to be dissolved by adding water thereto, and then vertically shaking the mixture 10 times. A time period required for the dissolution was less than 1 minute.

The purities of Compound A, oxidized form contents, and total analog contents at the respective time points of the respective formulations were as shown in the following table. In the table, the column "Lyophilization solution" shows stability data on the lyophilization solutions on the day of their preparation, and the columns "Day 0" and "Day 15" show stability data on the lyophilized preparations on the day of their production and 15 days after the production, respectively.

**Table 9**

| Formulation | Purity (%) | | | Oxidized form (%) | | | Total analog (%) | | |
|---|---|---|---|---|---|---|---|---|---|
| | Lyophilization solution | Day 0 | Day 15 | Lyophilization solution | Day 0 | Day 15 | Lyophilization solution | Day 0 | Day 15 |
| 30 | 98.3 | 98.2 | 98.1 | 0.79 | 0.88 | 1.04 | 1.69 | 1.83 | 1.93 |
| 31 | 98.3 | 98.3 | 98.1 | 0.76 | 0.82 | 0.95 | 1.66 | 1.74 | 1.95 |

In each of the formulations 30 and 31, the oxidized form content at the time point of the preparation of the lyophilization solution was lower than that of the formulation 17. In addition, the amounts of the oxidized form at the time point of the preparation of the lyophilization solution and at the time point of the production of the lyophilized preparation were smaller than those of the formulations 20 to 29.

The following was effective in suppressing the production of the oxidized form: the lyophilization solutions and the lyophilized preparations were prepared and produced by using, for example, the PS20 solution and the buffer subjected to nitrogen bubbling in advance.

### [Test Example 6: Investigation 4 on Stability of Lyophilized Preparation of Compound A]

A suppressing effect on an oxidized form at the time point of the preparation of a lyophilization solution exhibited by the addition of methionine was evaluated as described below.

Methionine was added to the mixed solution of distilled water and PS20 to prepare a 20 wt% aqueous solution of PS20 (containing 3 wt%, 2 wt%, 1 wt%, 0.2 wt%, or 0.1 wt% of methionine). Compound A was weighed in a 3-milliliter brown glass vial, and the above-mentioned 20 wt% aqueous solution of PS20 was added thereto so that the concentration of Compound A was 7.30 mg/g. The vial was sealed, and the mixture was stirred under room temperature and light shielding overnight. Thus, Compound A was dissolved. A dissolving liquid containing various additives, the liquid having been subjected to nitrogen bubbling for 20 minutes, was added to the above-mentioned solution of Compound A, and the contents were mixed by ultrasonic vibration for 1 minute. Thus, lyophilization solutions having compositions shown in the following table were prepared. In the table, the concentrations of Compound A are measured values.

**Table 10**

| Formulation | Composition of lyophilization solution | | | | | | |
|---|---|---|---|---|---|---|---|
| | Compound A (µg/mL) | PS20 (mg/mL) | NaH₂PO₄ (mg/mL) | Na₂HPO₄ (mg/mL) | Trehalose (mg/mL) | Sucrose (mg/mL) | Methionine (mg/mL) |
| 32 | 162.4 | 4 | 3.3 | 5.35 | 100 | - | 0.4 |
| 33 | 150.7 | 4 | 1.27 | 2.68 | - | 50 | 0.6 |
| 34 | 150.0 | 4 | 1.27 | 2.68 | - | 50 | 0.4 |
| 35 | 146.4 | 4 | 1.27 | 2.68 | - | 50 | 0.2 |
| 36 | 150.0 | 4 | 1.27 | 2.68 | - | 50 | 0.04 |
| 37 | 150.0 | 4 | 1.27 | 2.68 | - | 50 | 0.02 |

Each of the lyophilization solutions was loaded in an amount of 1 mL per 10-milliliter brown vial (Daiwa Special Glass Co., Ltd.), and a laminate-coated rubber stopper (Daikyo Seiko, Ltd.) was mounted on the vial, followed by freezing at - 35°C or less. After a pressure in each of the vials had been reduced, a temperature therein was increased to -10°C over 4 hours, and the frozen product was primarily dried for 32 hours. The temperature was increased to 20°C over 2 hours, and the dried product was secondarily dried for 2 hours. The vial was hermetically sealed with the rubber stopper while a vacuum state therein was kept, followed by capping with an aluminum cap. Thus, lyophilized preparations were obtained (formulations 32 to 37).

Each of the preparations having the formulations 32 to 37 was loaded into Aluminum Lamizip, and was stored under room-temperature and light-shielding conditions. The purities of Compound A, the contents of an oxidized form serving as the main decomposition product of Compound A, and total analog contents at the time point of the preparation of each of the formulations and at the time point of the storage thereof were calculated by HPLC measurement (area normalization). A test solution for measuring the purity of a lyophilized preparation was prepared by adding 1 mL of distilled water, and vertically shaking the mixture 10 times. Each of the formulations showed satisfactory water solubility, and was dissolved within 1 minute after water had been added thereto, and the mixture had been vertically shaken 10 times.

The purities of Compound A, oxidized form contents, and total analog contents at the respective time points of the respective formulations were as shown in the following table. In the table, the column "Lyophilization solution" shows stability data on the lyophilization solutions on the day of their preparation, and the columns "Day 0", "Day 14", and "Day 15" show stability data on the lyophilized preparations on the day of their production, and 14 days and 15 days after the production, respectively.

**Table 11**

| Formulation | Purity (%) | | | | Oxidized form (%) | | | | Total analog (%) | | | |
|---|---|---|---|---|---|---|---|---|---|---|---|---|
| | Lyophilization solution | Day 0 | Day 14 | Day 15 | Lyophilization solution | Day 0 | Day 14 | Day 15 | Lyophilization solution | Day 0 | Day 14 | Day 15 |
| 32 | 98.8 | 98.8 | - | 98.5 | 0.19 | 0.26 | - | 0.41 | 1.17 | 1.14 | - | 1.50 |
| 33 | 99.0 | 99.1 | 98.8 | - | 0.17 | 0.19 | 0.30 | - | 1.01 | 1.13 | 1.22 | - |
| 34 | 98.9 | 99.0 | 98.6 | - | 0.21 | 0.24 | 0.36 | - | 1.11 | 1.12 | 1.40 | - |
| 35 | 98.9 | 98.9 | 98.7 | - | 0.24 | 0.26 | 0.37 | - | 1.14 | 1.32 | 1.31 | - |
| 36 | 98.7 | 98.8 | 98.5 | - | 0.37 | 0.39 | 0.51 | - | 1.33 | 1.24 | 1.52 | - |
| 37 | 98.8 | 98.8 | 98.5 | - | 0.39 | 0.41 | 0.52 | - | 1.24 | 1.00 | 1.48 | - |

In the formulation 32, the oxidized form content at the time point of the preparation of the lyophilization solution was lower than those of the formulations 15 to 19, and hence the addition of methionine was effective in suppressing the production of the oxidized form.

Also in each of the formulations 33 to 37, the addition of methionine suppressed the production of the oxidized form at the time point of the preparation of the lyophilization solution. The production amount of the oxidized form depended on the addition amount of methionine. In each of the formulations 33 to 37, the addition weight of methionine corresponds to from 0.13 times to 3.98 times as large as that of Compound A.

### [Test Example 7: Investigation 5 on Stability of Lyophilized Preparation of Compound A]

The stability of each of lyophilized preparations including various excipients was investigated. Trehalose, sucrose, or mannitol was used as an excipient. In addition, a formulation having added thereto sodium chloride was investigated as an excipient-free formulation.

Methionine was added to the mixed solution of distilled water and PS20 to prepare a 20 wt% aqueous solution of PS20 (containing 2 wt% of methionine). Compound A was weighed in a 3-milliliter brown glass vial, and the above-mentioned 20 wt% aqueous solution of PS20 was added thereto so that the concentration of Compound A was 7.30 mg/g. The vial was sealed, and the mixture was stirred under room temperature and light shielding overnight. Thus, Compound A was dissolved. A dissolving liquid containing various additives, the liquid having been subjected to nitrogen bubbling for 20 minutes, was added to the resultant solution of Compound A, and each component in the mixture was dissolved by ultrasonic vibration for 1 minute. Thus, lyophilization solutions having compositions shown in the following table were prepared. In the table, the concentrations of Compound A are measured values.

**Table 12**

| Formulation | Composition of lyophilization solution | | | | | | | | |
|---|---|---|---|---|---|---|---|---|---|
| | Compound A (µg/mL) | PS20 (mg/mL) | NaH₂PO₄ (mg/mL) | Na₂HPO₄ (mg/mL) | Trehalose (mg/mL) | Sucrose (mg/mL) | Mannitol (mg/mL) | NaCl (mg/mL) | Methionine (mg/mL) |
| 38 | 146.4 | 4 | 2.54 | 5.35 | 50 | 0 | 0 | 0 | 0.4 |
| 39 | 144.8 | 4 | 1.27 | 2.68 | 0 | 50 | 0 | 0 | 0.4 |
| 40 | 145.5 | 4 | 1.27 | 2.68 | 0 | 0 | 50 | 0 | 0.4 |
| 41 | 145.3 | 4 | 1.27 | 2.68 | 0 | 0 | 0 | 78 | 0.4 |

Each of the lyophilization solutions was loaded in an amount of 1 mL per 10-milliliter brown vial (Daiwa Special Glass Co., Ltd.), and a laminate-coated rubber stopper (Daikyo Seiko, Ltd.) was mounted on the vial, followed by freezing at - 35°C or less. After a pressure in each of the vials had been reduced, a temperature therein was increased to -10°C over 4 hours, and the frozen product was primarily dried for 32 hours. The temperature was increased to 20°C over 2 hours, and the dried product was secondarily dried for 2 hours. The vial was hermetically sealed with the rubber stopper while a vacuum state therein was kept, followed by capping with an aluminum cap. Thus, lyophilized preparations were obtained (formulations 38 to 41).

Each of the preparations having the formulations 38 to 41 was loaded into Aluminum Lamizip, and was stored under room-temperature and light-shielding conditions. The purities of Compound A, the contents of an oxidized form serving as the main decomposition product of Compound A, and total analog contents at the time point of the preparation of each of the formulations and at the time point of the storage thereof were calculated by HPLC measurement (area normalization). A test solution for measuring the purity of a lyophilized preparation was prepared by adding 1 mL of distilled water, and vertically shaking the mixture 10 times. Each of the formulations showed satisfactory water solubility, and was dissolved within 1 minute after water had been added thereto, and the mixture had been vertically shaken 10 times.

The purities of Compound A, oxidized form contents, and total analog contents at the respective time points of the respective formulations were as shown in the following table. In the table, the column "Lyophilization solution" shows stability data on the lyophilization solutions on the day of their preparation, and the columns "Day 0" and "Day 7" show stability data on the lyophilized preparations on the day of their production and 7 days after the production, respectively.

**Table 13**

| Formulation | Purity (%) | | | Oxidized form (%) | | | Total analog (%) | | |
|---|---|---|---|---|---|---|---|---|---|
| | Lyophilization solution | Day 0 | Day 7 | Lyophilization solution | Day 0 | Day 7 | Lyophilization solution | Day 0 | Day 7 |
| 38 | 99.0 | 98.9 | 98.9 | 0.18 | 0.23 | 0.26 | 1.01 | 1.06 | 1.12 |
| 39 | 98.9 | 99.0 | 98.9 | 0.18 | 0.20 | 0.24 | 1.07 | 1.04 | 1.12 |
| 40 | 99.0 | 98.9 | 98.9 | 0.2 | 0.24 | 0.26 | 1.03 | 1.09 | 1.14 |
| 41 | 98.8 | 98.5 | 98.7 | 0.2 | 0.26 | 0.28 | 1.18 | 1.52 | 1.28 |

As shown in the foregoing table, the production of the oxidized form at the time of the preparation of the lyophilization solution or at the time of the storage of the lyophilized preparation was slight irrespective of the kind of an excipient, and whether or not the excipient was added. Various excipients may be used in the lyophilized preparation of Compound A, and a preparation to which no excipient is added may be produced.

### [Test Example 8: Investigation on Reconstitution of Lyophilized Preparation of Compound A]

Physiological saline (physiological salt solution "OTSUKA NORMAL SALINE") was added to a lyophilized preparation produced according to the same formulation as the formulation 32 except the concentration of Compound A in the lyophilization solution, and the preparation was dissolved in the saline so that a liquid amount was 5 mL. Thus, a reconstituted solution (liquid preparation) of Compound A was obtained. The concentration of Compound A in the reconstituted solution was calculated by HPLC measurement, and the content of Compound A was calculated. The results are shown in the following table. In the table, the content of Compound A is a ratio with respect to the content of Compound A in a lyophilization solution (measured value of the concentration of Compound A: 149.2 µg/mL) used in the production of the lyophilized preparation.

**Table 14**

| Liquid amount of reconstituted solution (mL) | Final concentration of Compound A | | Content of Compound A (%) |
|---|---|---|---|
| | (µg/mL) | (µM) | |
| 5 | 29.9 | 47.2 | 100.1 |

The reconstituted solution having the concentration of Compound A that an administration liquid was required to have was obtained by dissolving the lyophilized preparation in the physiological saline. The content of Compound A in the reconstituted solution was substantially the same as the content of Compound A in the lyophilization solution for producing the lyophilized preparation, wherein the volume of the lyophilization solution was the same as that of the reconstituted solution. In addition, the reconstituted solution was clear and free of visible particles.

### [Test Example 9: Investigation on Stability and Reconstitution of Lyophilized Preparation of Compound A]

A lyophilized preparation including PS40 or PS80 as a solubilizing agent was produced, and was evaluated for its stability and reconstitution.

Methionine was added to the mixed solution of distilled water and PS40 or PS80 to prepare a 20 wt% aqueous solution of PS40 and a 20 wt% aqueous solution of PS80 (each containing 2 wt% of methionine). Compound A was weighed in a 3-milliliter brown glass vial, and the 20 wt% aqueous solution of PS40 or the 20 wt% aqueous solution of PS80 described above was added thereto so that the concentration of Compound A was 7.30 mg/g. The vial was sealed, and the mixture was stirred under room temperature and light shielding overnight. Thus, Compound A was dissolved. A dissolving liquid containing various additives was added to the resultant solution of Compound A, and each component in the mixture was dissolved by ultrasonic vibration for 1 minute. Thus, lyophilization solutions having compositions shown in the following table were prepared. In the table, the concentrations of Compound A are measured values.

**Table 15**

| Formulation | Composition of lyophilization solution | | | | | | |
|---|---|---|---|---|---|---|---|
| | Compound A (µg/mL) | PS40 (mg/mL) | PS80 (mg/mL) | NaH₂PO₄ ·2H₂O (mg/mL) | Na₂HPO₄·12H₂O (mg/mL) | Sucrose (mg/mL) | Methionine (mg/mL) |
| 42 | 152.2 | 4 | 0 | 1.65 | 6.75 | 50 | 0.4 |
| 43 | 146.1 | 0 | 4 | 1.65 | 6.75 | 50 | 0.4 |

Each of the lyophilization solutions was loaded in an amount of 1 mL per 10-milliliter brown vial (Daiwa Special Glass Co., Ltd.), and a laminate-coated rubber stopper (Daikyo Seiko, Ltd.) was mounted on the vial, followed by freezing at - 35°C or less. After a pressure in each of the vials had been reduced, a temperature therein was increased to -10°C over 4 hours, and the frozen product was primarily dried for 32 hours. The temperature was increased to 20°C over 2 hours, and the dried product was secondarily dried for 2 hours. The vial was hermetically sealed with the rubber stopper while a vacuum state therein was kept, followed by capping with an aluminum cap. Thus, lyophilized preparations were obtained (formulations 42 and 43).

Each of the preparations having the formulations 42 and 43 was loaded into Aluminum Lamizip, and was stored under room-temperature and light-shielding conditions. The purities of Compound A, the contents of an oxidized form serving as the main decomposition product of Compound A, and total analog contents at the time point of the preparation of each of the formulations and at the time point of the storage thereof were calculated by HPLC measurement (area normalization). A test solution for measuring the purity of a lyophilized preparation was prepared by adding 1 mL of distilled water to the preparation, and vertically shaking the mixture 10 times. Each of the formulations showed satisfactory water solubility, and was dissolved within 1 minute after water had been added thereto, and the mixture had been vertically shaken 10 times.

The purities of Compound A, oxidized form contents, and total analog contents at the respective time points of the respective formulations were as shown in the following table. In the table, the column "Lyophilization solution" shows stability data on the lyophilization solutions on the day of their preparation, and the columns "Day 0" and "Day 7" show stability data on the lyophilized preparations on the day of their production and 7 days after the production, respectively.

**Table 16**

| Formulation | Purity (%) | | | Oxidized form (%) | | | Total analog (%) | | |
|---|---|---|---|---|---|---|---|---|---|
| | Lyophilization solution | Day 0 | Day 7 | Lyophilization solution | Day 0 | Day 7 | Lyophilization solution | Day 0 | Day 7 |
| 42 | 98.6 | 98.7 | 98.6 | 0.33 | 0.39 | 0.51 | 1.36 | 1.27 | 1.42 |
| 43 | 97.4 | 97.4 | 97.0 | 1.63 | 1.73 | 2.05 | 2.58 | 2.56 | 2.98 |

As shown in the foregoing table, even when PS40 or PS80 was used as a solubilizing agent, the production of the oxidized form at the time of the preparation of the lyophilization solution or at the time of the storage of the lyophilized preparation was slight. Various solubilizing agents may be used in the lyophilized preparation according to the embodiment of the present invention.

Distilled water was added to each of the formulations 42 and 43 to dissolve the formulation so that a liquid amount was 2 mL. Thus, a reconstituted solution (liquid preparation) of Compound A was obtained. The concentration of Compound A in the reconstituted solution was calculated by HPLC measurement, and the content of Compound A was calculated. The results are shown in the following table. In the table, the contents of Compound A are each a ratio with respect to the content of Compound A in the lyophilization solution used in the production of the lyophilized preparation.

**Table 17**

| Formulation | Liquid amount of reconstituted solution (mL) | Final concentration of Compound A | | Content of Compound A (%) |
|---|---|---|---|---|
| | | (µg/mL) | (µM) | |
| 42 | 2 | 75.4 | 119.2 | 99.1 |
| 43 | 2 | 73.4 | 116.0 | 100.5 |

The reconstituted solution of Compound A was obtained by dissolving the lyophilized preparation having each of the formulation 42 and the formulation 43 in the distilled water. The content of Compound A in the reconstituted solution was substantially the same as the content of Compound A in the lyophilization solution for producing the lyophilized preparation, wherein the volume of the lyophilization solution was the same as that of the reconstituted solution. In addition, the reconstituted solution was clear and free of visible particles.

### Industrial Applicability

The present invention can be suitably used in a medical field, for example, in the diagnosis of glaucoma or a cancer.

## Claims

1. A lyophilized preparation, comprising a compound represented by the following general formula (I) or a salt thereof: 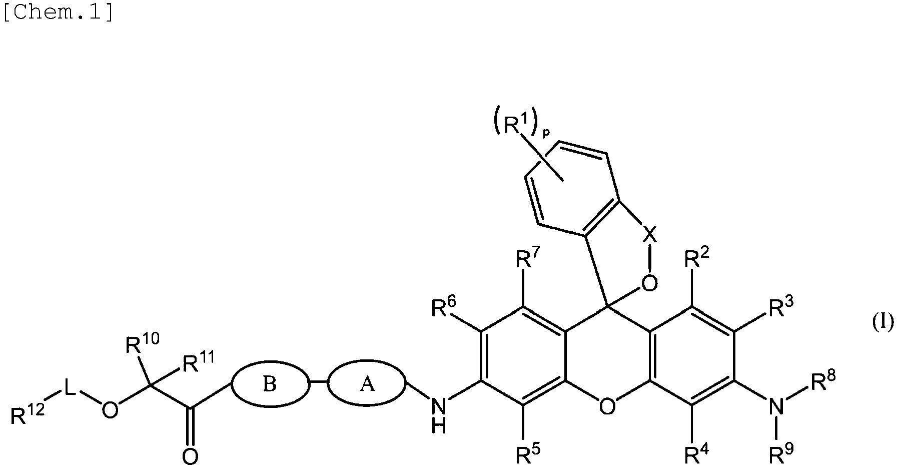 where:
A and B each independently represent an amino acid residue, and the amino acid residues are identical to or different from each other,
where a bond between A and NH bonded to A is an amide bond between a C-terminal of A and the NH, A and B are bonded through an amide bond, and a bond between B and a carbonyl group (C=O) bonded to B is an amide bond between an N-terminal of B and the carbonyl group (C=O);
R¹s are identical to or different from each other, and each represent a substituent bonded to a benzene ring;
"p" represents an integer of from 0 to 4;
R², R³, R⁴, R⁵, R⁶, and R⁷ each independently represent a hydrogen atom, a hydroxyl group (OH group), a halogen atom, or a linear, branched, or cyclic alkyl group that may be substituted;
R⁸ and R⁹ each independently represent a hydrogen atom, or a linear, branched, or cyclic alkyl group that may be substituted, or R⁸ and R⁹ may be bonded to form a ring, or R³ and R⁸ may be bonded to form a ring, and/or R⁴ and R⁹ may be bonded to form a ring;
X represents a linear or branched C1 to C3 alkylene group;
R¹⁰ and R¹¹ each independently represent a hydrogen atom, a hydroxyl group (OH), an alkoxy group, or a linear, branched, or cyclic alkyl group that may be substituted, or R¹⁰ and R¹¹ may be bonded to form a ring;
R¹² represents a hydrogen atom, a linear, branched, or cyclic alkyl group that may be substituted, a linear or branched alkenyl group that may be substituted, a linear or branched alkynyl group that may be substituted, an aryl group that may be substituted, or a heterocyclic group that may be substituted, or R¹² may be bonded to R¹⁰ or R¹¹ to form a ring; and
L represents an amide bond, an ester bond, or -(linear or branched alkylene-O-)ₙ- where "n" represents an integer of from 1 to 10, or L represents a single bond.

2. The lyophilized preparation according to claim 1, further comprising a solubilizing agent.

3. The lyophilized preparation according to claim 2, wherein the solubilizing agent comprises a polyoxyethylene sorbitan fatty acid ester and/or polyoxyethylene polyoxypropylene glycol.

4. The lyophilized preparation according to claim 2, wherein the solubilizing agent is at least one kind selected from polysorbate 20, polysorbate 40, and polysorbate 80.

5. The lyophilized preparation according to claim 2, wherein a content of the solubilizing agent is from 3 parts by weight to 300 parts by weight with respect to 1 part by weight of the compound or the salt thereof.

6. The lyophilized preparation according to claim 1, further comprising an antioxidant.

7. The lyophilized preparation according to claim 6, wherein the antioxidant comprises methionine.

8. The lyophilized preparation according to claim 6, wherein a content of the antioxidant is from 0.01 part by weight to 100 parts by weight with respect to 1 part by weight of the compound or the salt thereof.

9. The lyophilized preparation according to claim 1, further comprising at least one kind of additive selected from a pH regulator, an excipient, and a tonicity-adjusting agent.

10. The lyophilized preparation according to claim 1, wherein the lyophilized preparation is a glaucoma diagnostic agent or a cancer diagnostic agent.

11. A lyophilization solution, comprising:
a compound represented by the following general formula (I) or a salt thereof;
a solubilizing agent; and
water: 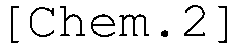 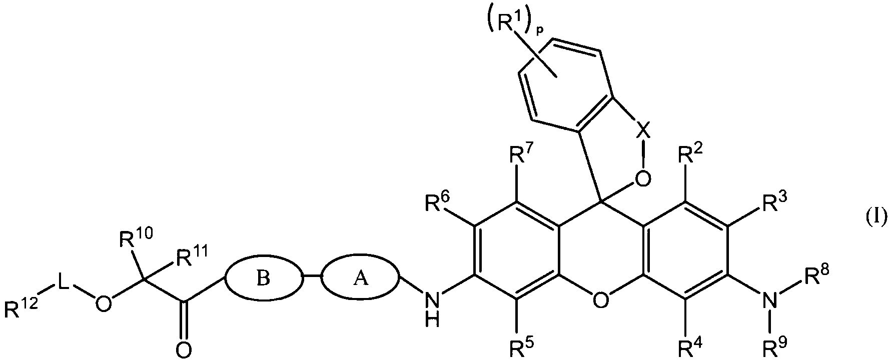 where:
A and B each independently represent an amino acid residue, and the amino acid residues are identical to or different from each other,
where a bond between A and NH bonded to A is an amide bond between a C-terminal of A and the NH, A and B are bonded through an amide bond, and a bond between B and a carbonyl group (C=O) bonded to B is an amide bond between an N-terminal of B and the carbonyl group (C=O);
R¹s are identical to or different from each other, and each represent a substituent bonded to a benzene ring;
"p" represents an integer of from 0 to 4;
R², R³, R⁴, R⁵, R⁶, and R⁷ each independently represent a hydrogen atom, a hydroxyl group (OH group), a halogen atom, or a linear, branched, or cyclic alkyl group that may be substituted;
R⁸ and R⁹ each independently represent a hydrogen atom, or a linear, branched, or cyclic alkyl group that may be substituted, or R⁸ and R⁹ may be bonded to form a ring, or R³ and R⁸ may be bonded to form a ring, and/or R⁴ and R⁹ may be bonded to form a ring;
X represents a linear or branched C1 to C3 alkylene group;
R¹⁰ and R¹¹ each independently represent a hydrogen atom, a hydroxyl group (OH), an alkoxy group, or a linear, branched, or cyclic alkyl group that may be substituted, or R¹⁰ and R¹¹ may be bonded to form a ring;
R¹² represents a hydrogen atom, a linear, branched, or cyclic alkyl group that may be substituted, a linear or branched alkenyl group that may be substituted, a linear or branched alkynyl group that may be substituted, an aryl group that may be substituted, or a heterocyclic group that may be substituted, or R¹² may be bonded to R¹⁰ or R¹¹ to form a ring; and
L represents an amide bond, an ester bond, or -(linear or branched alkylene-O-)ₙ- where "n" represents an integer of from 1 to 10, or L represents a single bond.

12. The lyophilization solution according to claim 11, wherein the solubilizing agent comprises a polyoxyethylene sorbitan fatty acid ester and/or polyoxyethylene polyoxypropylene glycol.

13. The lyophilization solution according to claim 12, wherein the solubilizing agent is at least one kind selected from polysorbate 20, polysorbate 40, and polysorbate 80.

14. The lyophilization solution according to claim 11, further comprising an antioxidant.

15. The lyophilization solution according to claim 14, wherein the antioxidant comprises methionine.

16. The lyophilization solution according to claim 11, wherein a concentration of the compound or the salt thereof is 6 µM or more.

17. The lyophilization solution according to claim 11, further comprising at least one kind of additive selected from a pH regulator, an excipient, and a tonicity-adjusting agent.

18. A method of producing the lyophilization solution of claim 11, comprising:
mixing the compound or the salt thereof and the solubilizing agent or an aqueous solution thereof to prepare a concentrated solution; and
mixing the concentrated solution and a diluent.

19. The method of producing the lyophilization solution according to claim 18, wherein a concentration of the solubilizing agent in the concentrated solution is 5 wt% or more.

20. The method of producing the lyophilization solution according to claim 18, wherein the concentrated solution and/or the diluent contains an antioxidant.

21. The method of producing the lyophilization solution according to claim 18, further comprising subjecting the solubilizing agent or the aqueous solution thereof, the concentrated solution, and/or the diluent to deoxygenation treatment.

22. A method of producing a lyophilized preparation, comprising subjecting the lyophilization solution of any one of claims 11 to 17 to lyophilization treatment.

23. A liquid preparation, which is obtained by reconstituting the lyophilized preparation of any one of claims 1 to 10.
